# EUROPEAN PATENT SPECIFICATION

(11) **EP 0 353 971 B1**
(45) Date of publication and mention of the grant of the patent: **07.02.1996**
(21) Application number: 89307752.9
(22) Date of filing: 31.07.1989
(51) Int. Cl.: C07D 219/04, C07F 9/64, G01N 33/53, C12Q 1/68

(54) **Acridinium esters and method for detection of an analyte using acridinium esters and liposomes**
Akridiniumester und Verfahren zum Nachweis von Analyt mit Akridiniumestern und Liposomen
Esters d'acridinium et procédé pour la détection d'analyte utilisant des esters d'acridinium et des liposomes

(30) Priority: 01.08.1988 US 226639
(43) Date of publication of application: 07.02.1990
(73) Proprietor: Ciba Corning Diagnostics Corp., Medfield Massachusetts 02052 (US)
(72) Inventor: Law, Say-Jong, Westwood, MA 02090 (US); Piran, Uri, Sharon, MA 02067 (US)
(74) Representative: Froud, Clive

(56) References cited:
- EP-A- 0 082 636
- EP-A- 0 257 541
- EP-A- 0 263 657
- GB-A- 1 461 877

## Description

This invention relates to acridinium esters and to a method for detection of an analyte using acridinium esters and liposomes; more particularly, it relates to a method for the detection of an analyte in a fluid sample using acridinium esters encapsulated within the walls of liposomes (lumisomes), and to acridinium esters useful as chemiluminescent markers, which may be encapsulated within liposome vesicles without significant leakage of the esters from the vesicles.

The use of liposomes as carriers of marker molecules for nonisotopic immunoassays is known. (See, e.g.,U.S. Patent Nos.4,704,355; 4,695,554; 4,656,129 and 4,193,983.) An important advantage of using liposomes in immunoassays is the ability of liposomes to carry a large number of marker molecules per liposome vesicle, and thereby provide an amplified signal to immunoassays. Immunoassays utilizing liposomes with encapsulated macromolecular markers, such as enzymes, or small organic marker molecules, such as fluorescent or absorbing dyes, spin-labels, metal chelators, and enzyme activators or inhibitors, have been described.(See, e.g., Cricka, L., and Carter, T., Clinical and Biochemical Luminescence, pp. 153-178 (Marcel Dekker, Inc., New York and Basel, 1982).)

Prior to the present invention, chemiluminescent markers, such as the acridinium esters described in Ann. Clin. Biochem. 25, p. 27, (1988); Clin. Chem. 31, p. 664, (1985); EP-A-82,636, and U.S. Patent No. 4,745,181, have only been used as labels for immunoassays by conjugating them directly to biological molecules, such as antigens or antibodies. The lipophilic nature of the prior art acridinium esters and other chemiluminescent compounds renders them unsuitable for encapsulation within liposomes because of their rapid leakage through the liposome wall. Additionally, the limited water-solubility of prior art acridinium esters and other chemiluminescent compounds only allows the encapsulation of a few marker molecules per liposome vesicle, resulting in relatively low signal amplification.

Accordingly, it is an object of the present invention to provide a method for detecting an analyte using acridinium esters. It is also an object of the present invention to provide hydrophilic acridinium esters useful for encapsulation within liposomes for use as chemiluminescent markers.

The present invention provides a liposome characterised in that it comprises an acridinium ester encapsulated therein. (The term "lumisome" is used herein.)

Such lumisomes preferably comprise the acridinium esters also provided by the present invention which correspond to the following general formula: 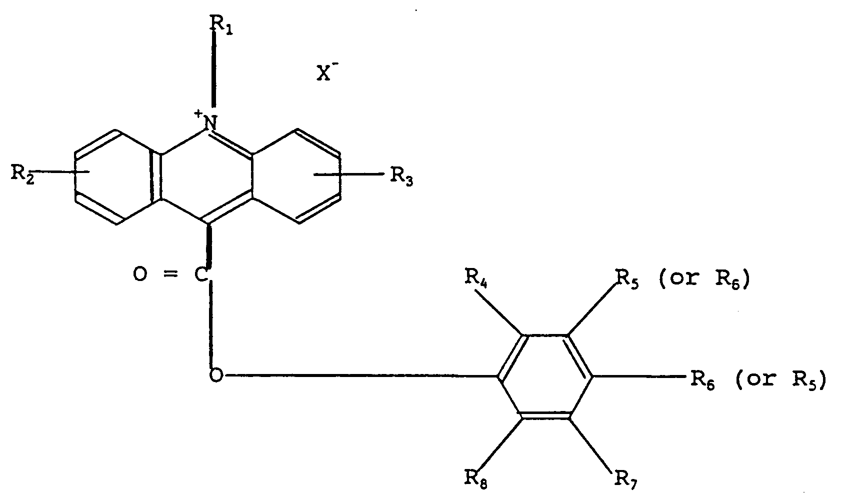
wherein
R₁ represents alkyl, alkenyl, alkynyl, aryl or aralkyl, which may contain one or more heteroatoms;
R₂, R₃, R₅ and R₇ independently represent hydrogen, amino, alkoxyl, hydroxyl, -COOH, halide, nitro, -CN, -SO₃H, -NHC(O)R, -C(O)R, -C(O)OR, -C(O)NHR or -SCN; wherein R represents alkyl, alkenyl, alkynyl, aryl or aralkyl, which may contain one or more heteroatoms;
R₄ and R₈ independently represent hydrogen, alkyl, alkenyl, alkynyl, aralkyl or alkoxyl;
R₆ represents -COOH, -R-Iₙ or -Q-R-Iₙ; wherein R represents a diradical derived from alkyl, alkenyl, alkynyl, aryl or aralkyl, which may contain one or more heteroatoms; Q represents -O-, -S-, -NH-, -C(O)-. -SO₃, diazo, -NHC(S)NH-, -NHC(O)NH-, -NHC(O)O-, -C(O)NH- or -NHC(N⁺H₂)-; I represents -SO₃H, -OSO₃H, -PO(OH)₂, -OPO(OH)₂ or -COOH; and n represents an integer of at least 1:
and
X represents an anion.

Certain preferred embodiments of the present invention are as follows: wherein R₁ and R may contain up to 24 carbon atoms and/or may contain up to 20, preferably up to 10, heteroatoms, which may be selected from nitrogen, oxygen, phosphorus and sulfur; R₁ preferably represents C₁-C₁₀ alkyl; and/or wherein R₂, R₃, R₅ and R₇ independantly represent hydrogen, amino, -COOH, -CN, hydroxyl, C₁-C₄ alkoxyl, nitro, halide, -SO₃H or -SCN; preferably hydrogen, C₁-C₄ alkoxyl, nitro, amino or -SO₃H; and/or wherein X represent halide, CH₃SO₄⁻, FSO₃⁻, CF₃SO₃⁻, C₄F₉SO₃⁻ or OSO₂-Ph-CH₃; preferably halide; and/or wherein Q represents -O-, -S-, -NH-, -C(O)-, diazo, -NHC(S)NH-, -NHC(O)NH-, -NHC(O)-, -C(O)NH- or -NHC(N⁺H₂)-; and/or wherein R₄ and R₈ independently represent hydrogen, alkyl, alkenyl, alkynyl or alkoxyl, which may contain up to 8 carbon atoms; preferably C₁-C₄ alkyl; and/or wherein R₆ represents -Q-R-Iₙ; and/or wherein I represents -SO₃H, -OSO₃H, -PO(OH)₂, -OPO(OH)₂ or -COOH; and/or wherein n represents an integer of up to 20, preferably up to 10. More specifically, it is preferred that X represents bromide; R₁, R₄ and R₈ represent methyl; R₂, R₃, R₅ and R₇ represent hydrogen; and R₆ is selected from aminomethanesulfonic acid [also called sufomethylcarbamoyl, when joined to -CO-; see Example 2 below], 7-amino-1,3-naphthalenedisulfonic acid [also called N-7-(1,3-disulfonaphthalenyl) carbamoyl, when joined to -CO-; see Example 3 below], S-(3-sulfopropyl)cysteine [also called N-[1-carboxyl-2-(3-sulfopropylthio) ethyl] carbamoyl, when joined to -CO-; see Example 4 below], 2-aminoethyl hydrogen sulfate [also called N-(2-sulfonyloxyethyl) carbamoyl, when joined to -CO-; see Example 5 below], 2-aminoethylphosphonic acid [also called N-(2-phosphonoethyl) carbamoyl, when joined to -CO-; see Example 6 below], and 2-aminoethyl dihydrogen phosphate [also called N-(2-phosphonooxyethyl) carbamoyl, when joined to -CO-; see Example 7 below].

The present invention also provides a luminescent assay conjugate characterised in that it comprises such a lumisome coupled to at least one biologically-active molecule, which may be selected from antibodies, antigens, haptens and nucleic acid(s).

The present invention further provides a method for the determination of an analyte characterised in that it comprises combining a sample fluid, such a lumisome or such a conjugate and a first receptor for the analyte, and then determining the amount of lumisome label associated with the analyte; or, when the analyte is a member of a specific binding ligand/antiligand pair, detecting the value of the detectable lumisome label as compared to the determination of the detectable label of a sample with a known amount of analyte.

In such a method, for example, the analyte may be an antigen, a hapten, a DNA probe or a RNA probe and/or the receptor may be an antibody. As desired in such a method, the sample fluid is combined with a solid support comprising a second receptor for the analyte prior to, simultaneously with or after combining the sample fluid with the lumisome.

Referring to the accompanying illustrative drawings:
Figure 1 is a standard curve for Total T₄ Assay using lumisomes in accordance with the present invention.
Figure 2 is a standard curve for a Free T₄ Assay using lumisomes in accordance with the present invention.
Figure 3 is a standard curve for a CKMB Assay using lumisomes in accordance with the present invention.
Figure 4 is a comparison of two standard curves for a TSH Assay, the top curve representing a TSH assay using lumisomes in accordance with the present invention and the bottom curve representing a TSH assay using antibody directly labelled with acridinium ester.
Figure 5 is a comparison of two standard curves for a TSH Assay, the top curve representing a TSH assay using lumisomes in accordance with the present invention and shortened incubation times and the bottom curve representing a TSH assay using antibody directly labelled with acridinium ester and normal incubation times.

For the present purposes, these terms have the following meanings:
Analyte - the compound or composition to be measured which can be a ligand that is mono- or polyepitopic, antigenic, or haptenic. The analyte can be a piece of DNA or RNA.
Antigen - any substance capable of provoking an immune response in vertebrates, particularly with the production of specific antibodies.
Hapten - an incomplete antigen, incapable by itself to provoke an immune response but when suitably attached to another molecule becomes capable of producing antibodies which will specifically recognize the hapten.
Epitope - a specific chemical and spatial configuration which is specifically recognized by an antibody.
Ligand - any compound for which a receptor naturally exists or can be prepared.
Ligand analog - a modified ligand which can compete with the analogous ligand for a receptor, the modification providing means to join a modified ligand to another molecule.
Receptor - any compound capable of recognizing a particular spatial and polar organization of a molecule, i.e., epitopic site. Illustrative receptors include antibodies, enzymes, antibody fragments, such as Fab fragments, DNA or RNA fragments, lectins, complement components, conglutin, rheumatoid factors, hormones, avidin, staphylococcal protein A, and the like.
Antiligand - a receptor for a ligand.
DNA probe - piece of DNA that recognizes specific DNA or RNA sequences by hydridizing to complementary DNA or RNA.
RNA probe - piece of RNA that recognizes specific DNA or RNA sequences by hydridizing to complementary DNA or RNA.
Liposomes - single or multicompartmented bodies obtained when lipids, particularily lipid mixtures, are dispersed in aqueous suspension. The walls or membranes are composed of a continuous lipid bilayer.
Lumisomes - liposomes comprising an encapsulated acridinium ester.

The acridinium esters useful in the method of the present invention can be any acridinium ester which can be encapsulated within a liposome and which can generate a chemiluminescent signal.

An ionizable group for the present purposes is a functional group which retains a net positive or negative charge within a specific pH range. Preferably, the functional group will retain a net positive or negative charge within the pH range of from 2 to 10, more preferably from 5 to 9. I may represent various ionizable groups provided that the ionizable group is not deleterious to the encapsulation of the present acridinium ester within the liposome.

As indicated in the above general formula, the R₅ and R₆ positions may be interchanged in the acridinium esters according to the present invention.

The acridinium esters according to the present invention are highly soluble in water and may be encapsulated in liposomes at high concentrations. Once inside liposomes, the present acridinium esters remain encapsulated for extended periods of time and do not leak appreciably.

It will be appreciated by those skilled in the art that, although the acridinium esters in accordance with the present invention have been described with respect to their usefulness with liposomes, the present acridinium esters are also useful in other applications where acridinium esters are utilized, such as labelling ligands or analytes (such as antigens); labelling the specific binding partners of ligands or analytes (such as the corresponding antibodies); or labelling nucleic acids and molecules comprising nucleic acids.

Lumisomes useful in this invention can be prepared by any of the various known methods for producing either unilamellar liposome vesicles or multilamellar liposome vesicles. Lumisomes are single or multicompartmented bodies obtained when lipids or lipid mixtures are dispersed in an aqueous suspension containing the acridinium esters useful in this invention.

As an example of one method for producing lumisomes, lipids are dissolved in a suitable organic solvent, such as chloroform, and placed into a suitable vessel. A dry film of lipids is formed on the interior surface of the vessel by evaporation of the organic solvent. The aqueous solution containing the acridinium esters to be entrapped within the lumisomes is then placed in the vessel in contact with the lipid film. The lipid film is then dispersed in the aqueous solution by vigorous agitation or sonication.

Numerous other methods exist for forming liposomes which are useful for producing the lumisomes useful in this invention and it is left to the artisan to choose the method best suited for a desired use. A preferred method for manufacturing liposomes is disclosed in copending U.S. Patent 4902466, herein incorporated by reference.

The lumisome can be derivatized with a ligand, ligand analog, or anti-ligand using known procedures in the art. Depending on the intended use of the lumisome, the ligand, ligand analog, or anti-ligand can be an antigen, hapten, antibody, nucleic acid, DNA, RNA, avidin or other receptor.

The lumisomes so formed can be used as tracers in assays in which an analyte in a sample fluid is to be detected. The type of assay utilized and/or the analyte to be detected will determine the ligand, ligand analog or anti-ligand used to form the lumisome. For example, if a competitive assay is used for determining an antigen or hapten, the ligand or ligand analog employed will be either the analyte or its analog.

If a sandwich assay is to be used the ligand, ligand analog or anti-ligand employed would be specific for the analyte to be assayed. For example, an antibody, such as a monoclonal antibody, elicited in response to the analyte to be assayed, could be used to derivatize the lumisome.

An example of an assay for detecting a DNA or RNA probe using the lumisomes of this invention is as follows: The DNA or RNA probe is tagged with a ligand such as a hapten or a biotinylated modified nucleotide. The DNA or RNA probe is allowed to hybridize with complementary DNA or RNA and immobilized on a solid support. The immobilized probe is then reacted with lumisomes comprising a receptor for the ligand, such as an antibody or if the probe is biotinylated, avidin. The lumisomes are ruptured and the amount of signal generated by the encapsulated acridinium ester is measured.

Alternatively, a receptor can be utilized which recognizes and binds to the probe/complementary DNA or RNA hybrid, in the absence of a tag, such as an anti-hybrid antibody .

The following Examples are presented to illustrate the present invention.

### Example 1

### Preparation of 2′,6′-Dimethyl-4′-carboxylphenyl 10-methyl-acridinium-9-carboxylate bromide (DMAE-COOH)

A mixture of 2′,6′-dimethyl-4′-benzyloxycarbonylphenyl 10-methyl-acridinium-9-carboxylate methosulfate (7.9, 13.4 mmole) (prepared as described in U.S. Patent No. 4,745,181), 150 ml of glacial acetic acid and 46 ml of 48% hydrogen bromide was heated at 100°C-105°C for 3 hours and then left at room temperature overnight. 400 ml of anhydrous ethyl ether was added to the mixture to form a second mixture which was then refrigerated 3 days. The second mixture was then filtered to produce a yellow crystalline residue. The residue was washed with anhydrous ethyl ether and air dried.

### Example 2

### Preparation of 2′,6′-Dimethyl-4′-(sulfomethylcarbamoyl)phenyl 10-methyl-acridinium-9-carboxylate bromide (DMAE-AMS)

A solution of 2′6′-dimethyl-4′-carboxylphenyl 10-methy-acridinium-9-carboxylate bromide (DMAE-COOH, 20 mg, 0.043 mmole) in 2 ml of dimethylformamide (DMF)/CHCl₃ (1:1) was cooled in an ice bath, treated with triethylamine (31 ul, 0.215 mmole) and ethyl chloroformate (6.1 ul, 0.065 mmole) to form a reaction mixture. After 30 min. the reaction mixture was evaporated. The residue of the evaporation was reconstituted in 2 ml of DMF, treated with triethylamine (31 ul, 0.215 mmole) and aminomethanesulfonic acid (9.5 mg, 0.086 mmole) to form a second reaction mixture. The second reaction mixture was stirred at room temperature overnight, and evaporated. The crude product so formed was purified on one analytical TLC plate (Silica gel 60, F254, Merck & Co., Inc., Rahway, N.J.), and developed with chloroform/methanol/water (65:25:4). The yellow band which developed on the plate (Rf= 0.38) (which could also be detected under both long and short UV light) was stripped from the plate and eluted with the same solvent system. The eluent so produced was evaporated and the residue of the evaporation triturated with methanol to form a mixture. This mixture was then filtered through a polycarbonate membrane (13mm diameter, 0.2 um pore size) (Nucleopore Corp., Pleasanton, CA) mounted on a syringe filter holder, and the filtrate so produced was evaporated to give DMAE-AMS (15 mg, 62%).

### Example 3

### Preparation of 2′,6′-Dimethyl-4′-[N-7-(1,3-disulfonaphthalenyl)carbamoyl]phenyl 10-methyl-acridinium-9-carboxylate Bromide (DMAE-ANDS)

A solution of 2′,6′-dimethyl-4′-carboxylphenyl 10-methyl-acridinium-9-carboxylate bromide (DMAE-COOH, 18 mg, 0.038 mmole) in 3.6 ml of a dioxane/water (1:1) mixture was treated with 1-(3-dimethylaminopropyl)-3-ethylcarbodiimide hydrochloride (37 mg, 0.193 mmole, Aldrich Chemical Co., Inc., Milwaukee, WI) at room temperature for 5 minutes to form a reaction mixture. A solution of 7-amino-1,3-naphthalenedisulfonic acid (ANDS, 26 mg, 0.076 mmole, Aldrich Chemical Co., Inc.) in 0.9 ml of water was added to the reaction mixture which was then stirred at room temperature overnight and evaporated.

The residue of the evaporation was dissolved in a minimal amount of 0.1M sodium carbonate to obtain a solution at neutral pH. This solution was mixed with an equal amount of methanol and purified on a 20x20cm preparative TLC plate (Silica gel 60, F254, Merck & Co., Inc.), and developed with chloroform/methanol/water (55:40:5). The yellow band which developed (Rf = 0.4) was treated in the same manner as the yellow band described in Example 1 to give DMAE-ANDS (15 mg, 50%). Fast Atom, Bombardment (FAB) Mass Spectral Analysis (by Oneida Research Services, Whitesboro, N.Y.) in the positive ion mode gave a M+ peak of 671, a M+Na peak of 693, and a M+2Na peak of 715. A bromide peak of 80 was detected in the negative ion mode.

### Example 4

### Preparation of 2′,6′-Dimethyl-4′-{N-[1-carboxyl-2-(3-sulfopropylthio)ethyl]carbamoyl}phenyl 10-methyl-acridinium-9-carboxylate bromide (DMAE-SCYS)

A solution of 2′,6′-dimethyl-4′-carboxylphenyl 10-methyl-acridinium-9-carboxylate bromide (DMAE-COOH, 50 mg, 0.107 mmole) in 10 ml of DMF/CHCl₃ (1:1) was cooled in an ice bath, treated with triethylamine (77 u., 0.535 mmole) and ethyl chloroformate (20 ul, 0.214 mmole) to form a reaction mixture. After 30 min. the reaction mixture was evaporated. The residue of the evaporation was reconstituted in 10 ml of DMF/CHCl₃ (1:1), treated with triethylamine (77 u., 0.535 mmole), and S-3-sulfopropyl-L-cysteine (51 mg, 0.214 mmole) (prepared by the method of U.T. Ruegg and J. Rudinger, J. Peptide Protein Res. 6, 447, 1974) to form a second reaction mixture. The second reaction mixture was heated at 60°C-70°C overnight, and evaporated.

The residue of the evaporation was purified on one 20x20cm preparative TLC plate (Silica gel 60, F254, Merck & Co., Inc.), developed with chloroform/methanol/water (55:40:5). The yellow band which developed (Rf = 0.4) (which could also be detected under both long and short UV light) was treated in the same manner as the yellow band described in Example 1 to give the DMAE-SCys (37 mg, 36%).

FAB Mass Spectral Analysis in the positive ion mode gave a M+ peak of 611, a M+Na peak of 633, and a M+Na,K peak of 673.

### Example 5

### Preparation of 2′,6′-Dimethyl-4′-[N-(2-sulfonyloxyethyl)carbamoyl]phenyl 10-methyl-acridinium-9-carboxylate bromide (DMAE-AEOS)

A solution of 2′,6′-dimethyl-4′-carboxylphenyl 10-methyl-acridinium-9-carboxylate bromide (DMAE-COOH, 35 mg, 0.075 mmole) in 5 ml of DMF was cooled in an ice bath, treated with triethylamine (44 ul, 0.31 mmole) and ethyl chloroformate (8.5 ul, 0.090 mmole). After 20 minutes, aminoethyl hydrogen sulfate Aldrich, 30 mg, 0.21 mmole) was added to form a reaction mixture, and the ice bath removed. The reaction mixture was stirred at room temperature overnight and evaporated. The residue of the evaporation was triturated with 5 ml of a chloroform/methanol/water (73:24:3) mixture, and filtered to remove the insoluble materials. The filtrate so produced was concentrated, purified on a 20x20cm preparative TLC plate (Silica gel 60, F254, Merck & Co., Inc.) and developed with chloroform/methanol/water (65:25:4). The yellow band which developed (Rf = 0.48) (which could also be detected under both long and short UV light) was treated in the same manner as the yellow band described in Example 1 to give DMAE-AEOS (14 mg, 32%).

### Example 6

### Preparation of 2′,6′-Dimethyl-4′-[N-(2-phosphonoethyl)carbamoyl]phenyl 10-methyl-acridinium-9-carboxylate bromide (DMAE-AEP)

A solution of 2′,6′-dimethyl-4′-carboxylphenyl 10-methyl-acridinium-9-carboxylate bromide (DMAE-COOH, 100 mg, 0.215 mmole) in 20 ml of 25% DMF in chloroform was cooled in an ice bath, treated with triethylamine (180 ul, 1.30 mmole) and ethyl chloroformate (62 ul, 0.65 mmole) to form a reaction mixture. After 30 minutes, the reaction mixture was evaporated. The residue of the evaporation was reconstituted in 12 ml of DMF. To this solution so formed was added triethylamine (300 ul, 2.15 mmole) and 2-aminoethylphosphonic acid (80 mg, 0.64 mmole, Aldrich Chemical Co., Inc.) in 5 ml of water to form a second reaction mixture. The second reaction mixture was stirred at room temperature overnight and evaporated. The residue of the evaporation was taken up in 2-3 ml of chloroform/methanol/water (73:24:3), purified on one 20x20cm preparative TLC plate (Silica gel 60, F254, Merck & Co., Inc.) and developed with chloroform/methanol/water (65:25:4). The yellow band which developed on the plate (Rf = 0.45) (which can be detected also under long and short UV light) was treated in the same manner as the yellow band described in Example 1 to give DMAE-AEP (72 mg, 58%).

FAB Mass Spectral analysis in the positive ion mode gave a M+ peak of 493 and a M+Na peak of 515.

### Example 7

### Preparation of 2′,6′-Dimethyl-4′[N-(2-phosphonooxyethyl)carbamoyl]phenyl 10-methyl-acridinium-9-carboxylate bromide (DMAE-AEOP)

A solution of 2′,6′-dimethyl-4′-carboxylphenyl 10-methyl-acridinium-9-carboxylate bromide (DMAE-COOH, 100 mg., 0.215 mmole) in 20 ml of 25% DMF in chloroform was cooled in an ice bath, treated with triethylamine (180 ul, 1.30 mmole) and ethyl chloroformate (62 ul, 0.65 mmole) to form a reaction mixture. After 30 minutes, the reaction mixture was evaporated. The residue of the evaporation was reconstituted in 12 ml of DMF. To this solution so formed was added triethylamine (300 ul, 2.15 mmole) and 2-aminoethyl dihydrogen phosphate (91 mg, 0.64 mmole, Aldrich Chemical Co., Inc.) in 5 ml of water to form a second reaction mixture. The second reaction mixture was stirred at room temperature overnight and evaporated. The residue of this evaporation was taken up in 2-3 ml of chloroform/methanol/water (73:24:3), purified on one 20x20cm preparative TLC plate (Silica gel 60, F254, Merck & Co., Inc.) and developed with chloroform/methanol/water (65:25:4). The yellow band which developed (Rf = 0.45) (which could also be detected also under long and short UV light) was treated in the same manner as the yellow band described in Example 1 to give DMAE-AEOP. (100 mg, 79%) .

FAB Mass Spectral analysis in the positive ion mode gave a M+ peak of 509.

### Example 8

### Rate of Leakage of Acridinium Esters From Lumisomes

Lumisomes were prepared as follows:
Chloroform solutions containing 25 mg dipalmitoyl phosphatidylcholine, 13.5 mg cholesterol, and 2.2 mg dipalmitoyl phosphatidylglycerol were air-dried on a round flat glass dish, 7 cm in diameter, and were placed for 16 hours in a vacuum to produce dry lipid films. Acridinium esters DMAE-COOH, DMAE-AMS, and DMAE-ANDS, were each dissolved in a solution of 0.215M sucrose, 0.25mM EDTA, pH 7 (0.1-0.26mg/ml). Acridinium esters DMAE-SCYS, DMAE-AEOS, DMAE-AEP, and DMAE-AEOP, were each dissolved in a solution of 50mM sodium phosphate, pH 7.4 (0.1-0.26mg/ml). A three ml aliquot of each acridinium ester solution so formed was added to a separate dry lipid film and gently mixed at 45°C for 10 min. to produce a lumisome suspension. Each suspension so formed containing DMAE-COOH, DMAE-AMS, and DMAE-ANDS, was extruded through polycarbonate membranes with a pore size of 0.4 and 0.2 microns and then washed 4 times by ultracentrifugations at 45,000 rpm for 30 min. in TRIS Buffer (0.1M TRIS, 0.03M NaCl, 0.01% NaN₃, 0.5mM EDTA, pH 7.8) to produce a lumisome pellet. The lumisome pellets so formed were each resuspended in 10 ml of TRIS Buffer. Each lumisome suspension containing DMAE-SCYS, DMAE-AEOS, DMAE-AEP, and DMAE-AEOP, was extruded through polycarbonate membranes with a pore size of 0.4 and 0.2 microns and then washed 4 times by ultracentrifugation at 45,000 rpm for 30 min. in 50mM sodium phosphate, pH 7.4, to produce a lumisome pellet. The lumisome pellets so formed were each resuspended in 10 ml of 50mM sodium phosphate, pH 7.4. A sample of each resuspended lumisome pellet was mixed with 0.1% TRITON X-100 detergent (weight/volume) for determination of the total amount of encapsulated acridinium ester. Aliquots of each of the resuspended lumisome pellets were incubated for 7 days at 4°C and 37°C. The aliquots were then centrifuged at 45,000 rpm for 2 hours. After centrifugation, a sample was taken from each supernatant and mixed with 0.1% TRITON X-100. The concentration of acridinium ester in each supernatant was then measured using the Ciba Corning Magic^{R} LITE Analyzer (Ciba Corning Diagnostics Corp., Medfield, MA). The percent leakage of acridinium ester was then calculated by comparing the amount of acridinium ester in the supernatant to the total amount of acridinium ester originally encapsulated within the lumisomes.

**Table 1**

| Leakage of Acridinium Esters From Lumisomes | | |
|---|---|---|
| Acridinium Ester | % Leakage at 4°C/7 Days | % Leakage at 37°C/7 Days |
| DMAE-COOH | 25.0% | 36.0% |
| DMAE-AMS | 0.8% | 26.0% |
| DMAE-ANDS | 1.3% | 1.9% |
| DMAE-SCYS | 0.5% | 4.9% |
| DMAE-AEOS | 1.4% | 52.8% |
| DMAE-AEP | 0.3% | 1.0% |
| DMAE-AEOP | 1.8% | 1.7% |

### Example 9

### Effect of Different Buffers on Acridinium Ester Leakage From Lumisomes

Lumisomes encapsulating DMAE-ANDS were prepared according to the procedure described in Example 8 except that the DMAE-ANDS was dissolved in the four buffers listed in Table 2, prior to addition to the dry lipid film. The buffers were then used to wash, resuspend and store, respectively, the four lumisome prepartions in Table 2. The lumisomes so prepared were stored in their respective buffers for 35 days at either 4°C or 37°C and then centrifuged at 45,000 rpm for 2 hours. The concentration of DMAE-ANDS in each supernatant was then measured using a Ciba Corning Magic^{R} LITE Analyzer. The percent leakage of DMAE-ANDS was then calculated by comparing the amount of DMAE-ANDS in the supernatant versus the amount of DMAE-ANDS originally encapsulated within the lumisomes.

**Table 2**

| Effect of Different Buffers on the Leakage of DMAE-ANDS from Lumisomes After 35 Days at 4°C and at 37°C | | |
|---|---|---|
| Buffer (50mM) | % Leakage at 4°C | % Leakage at 37°C |
| Sodium phosphate pH 7.4 | 0.35% | 0.85% |
| Sodium acetate pH 7.4 | 0.33 | 1.39 |
| Sodium tartarate pH 7.4 | 0.62 | 1.30 |
| Sodium HEPES pH 7.4 | 0.79 | 1.71 |

### Example 10

### Total T₄ Assay

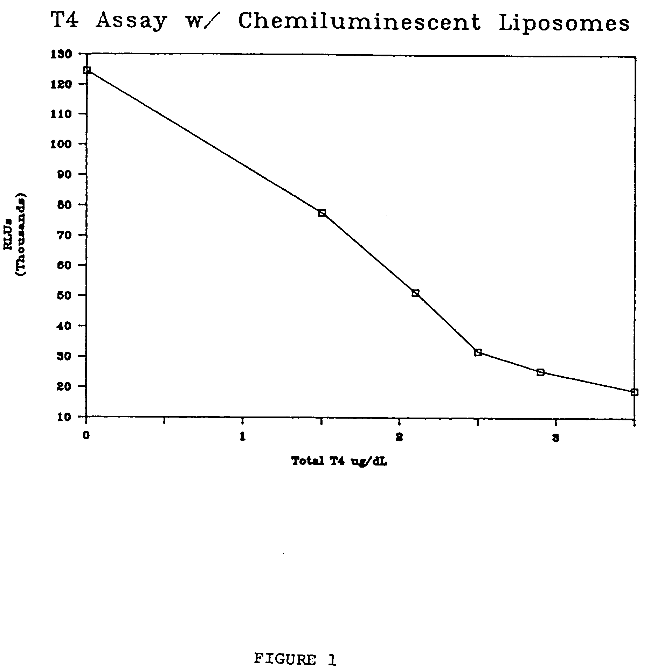

A. Reagent Preparation.
   Thyroxine (T₄) lumisomes encapsulating DMAE-AMS were prepared as described in Example 8 except that 0.16 mg of dipalmitoyl-phosphatidylethanolamine-succinyl-thyroxine (prepared as described in European Patent EP-B-307 175 herein incorporated by reference) was added to the lipid mixture. DMAE-AMS at 0.2 mg/ml in phosphate buffer was used for hydration of the lipid film. The final lumisome preparation was diluted in Buffer A (0.02M Tris, 0.1M NaCl, 0.001M EDTA, 0.1% bovine serum albumin (BSA), 0.1% sodium azide, pH 7.8).
   Monoclonal anti-T₄ antibody was produced in mice (A/J) by immunization with a BSA-T₄ conjugate and subsequent fusion of the splenocytes with Sp2/0-Ag 14 myeloma cells by the procedure described by Kohler and Milstein in Nature (London), Vol. 256, pp. 495-497 (1975). Hybridoma cells secreting anti-T₄ antibody were detected by radioimmunoassay using the following procedure: Supernatant from the cells were diluted 1:5 in phosphate buffered saline containing 0.1% (weight/volume) bovine gamma globulin. 100 ul of each diluted supernatant and 100 ul of ¹²⁵ I-labelled T₄ were added to a test tube and were incubated for one (1) hour at room temperature. Goat anti-mouse IgG coupled to paramagnetic particles were added to each tube for ten (10) minutes at room temperature. The particles were magnetically separated and counted. The cells that tested positive (i.e., produced counts over background) were plated at 0.1 cell/well and then retested after growth.
   Cells resulting from this regrowth which tested positive were then injected introperitoneally into pristane-primed mice (CAF). Ascitic fluid from these mice was collected after 3-5 weeks. The anti-T₄ antibody was purified from the ascitic fluid by Protein A column chromatography using the Affi-Gel Protein A MAPS II Kit (Bio-Rad Laboratories, Richmond, CA) according to the written protocol provided by the kit. The anti-T₄ antibody was immobilized on paramagnetic particles as described by Groman et al, BioTechniques 3:156-160 (1985). The antibody-derivatized particles were diluted to a concentration of 50 ug of particles per 0.1 ml of Buffer B (0.03M phosphate, 0.1M NaCl, 2.9 mg/ml merthiolate, and 0.1% BSA, 0.1% sodium azide, pH 7.4).
B. Assay procedure
   A series of standards (0.05 ml) with known increasing amounts of T₄ Were added to 12x75mm plastic tubes. 0.1 ml of the T₄-lumisomes prepared in A containing 10x10⁶ RLU (relative light units) in Buffer A were then added, followed by the addition of 0.5 ml of the paramagnetic-particles immobilized with monoclonal anti-T₄ antibody as prepared in A. The tubes were incubated for 1 hour at room temperature and then placed in a magnetic field of a specially designed rack useful for magnetic separation of paramagnetic particles in test tubes (available from Ciba Corning Diagnostics Corp., Medfield, MA). The magnetic field separated the particles from the supernatant and the supernatant was then decanted. The particles were washed once in 1 ml of phosphate-buffered saline, vortexed, and magnetically separated. The particles were resuspended in 0.1 ml of TRITON X-100, 0.1% (w/v) in deionized water.
   The tubes were then placed in a luminometer (MAGIC^{R} LITE Analyzer, Ciba Corning Diagnostics Corp., Medfield, MA). 0.3 ml of a solution of 0.1% hydrogen peroxide in 0.1N HNO₃ was added to each tube by the luminometer and the light emission was triggered by the addition of 0.3 ml of 0.25N NaOH containing ARQUAD surfactant.(Armark Chemicals, Chicago, Illinois). The measured RLU for each tube was plotted against its respective T₄ concentration as shown in Figure 1.

### Example 11

### Free T₄ Assay

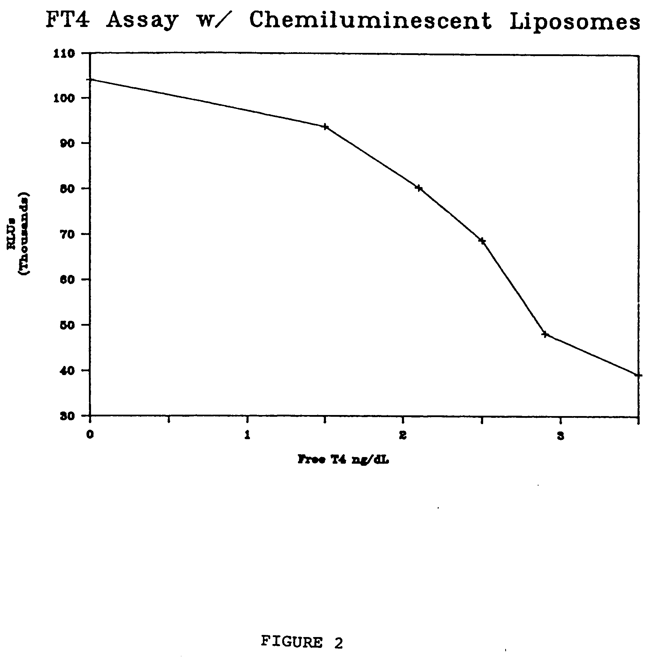

A series of serum-based standards containing known increasing concentrations of free T₄ were added to 12x75mm plastic tubes. 0.1 ml of the T₄-lumisomes prepared in Example 10A containing 10x10⁶ RLU was added, followed by the addition of 0.5 ml of the paramagnetic-particles immobilized anti-T₄ antibody as prepared in Example 10A, in Buffer B minus merthiolate. The reaction was carried out for 1 hour at room temperature and the tubes were further processed and read as in Example 10. Figure 2 shows the standard curve obtained by plotting the measured RLU for each tube against its respective free T₄ concentration.

### Example 12

### Creatine kinase MB (CKMB) Assay

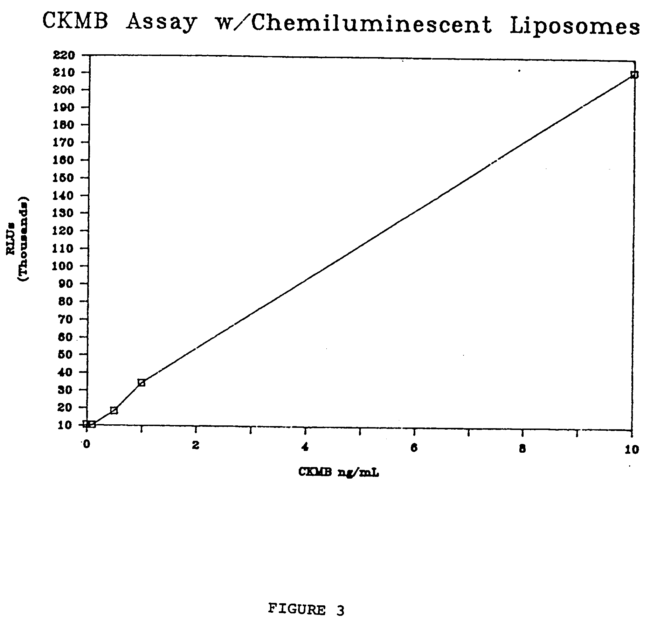

A. Reagent preparation
   Monoclonal antibodies to creatine kinase MB (CKMB) and creatine kinase BB (CKBB) were prepared as described by Piran et al, Clinical Chemistry 33:1517-1520 (1987).
   Lumisomes were prepared as described in Example 8, except that 1 mg dithiopyridyl dipalmitoyl phosphatidylethanolamine (DTP-DPPE), prepared according to Barbet et al, J. Supramolecular Structure 16:243-258 (1981), was added to the lipid mixture. The dry lipid film was hydrated with DMAE-ANDS, 2 mg/ml, and the final lumisome preparation was diluted in 0.1M sodium phosphate, 0.15M NaCl, and 0.005m EDTA, pH 7.5.
   The anti-CKMB monoclonal antibody was coupled to the DTP-DPPE containing lumisomes by the method of Barbet et al J. Supromolecular Structure 16:243-258 (1981). The anti-CKMB antibody bearing lumisomes were diluted in Buffer C (0.1M 1,4-piperazinediethanesulfonic acid, 0.15M NaCl, 0.001M EDTA, 0.1% NaN₃, 0.1% BSA, pH 6.5).
   The anti-CKBB monoclonal antibody was immobilized on paramagnetic particles as described in Example 10 for the anti-T₄ antibody and diluted in Buffer C to a final concentration of 100 ug of particles per ml of Buffer C.
B. Assay procedure
   A series of serum-based standards (0.1 ml) with known increasing concentrations of human cardiac CKMB were added to 12x75mm plastic tubes. 0.1 ml of the anti-CKMB antibody bearing lumisomes prepared in A containing 10x10⁶ RLU was added to each tube and the tubes were then incubated for 30 minutes at room temperature. To each tube was then added 50 ug of the anti-CKBB antibody bearing paramagnetic-particles prepared in A and the the tubes were incubated for another 30 minutes at room temperature. The tubes were then processed and read as in Example 10, except that Buffer C minus BSA was used in the wash step instead of phosphate-buffered saline. The measured RLU for each tube was plotted against its respective CKMB concentration as shown in Figure 3.
C. Four samples of human sera were assayed using the procedure described in B and concentrations of <1, 21, 24 and 68 ng/ml CKMB respectively, were determined. When these four samples were assayed using the CKMB Magic^{R} LITE Assay (Ciba Corning Diagnostics Corp., Medfield, MA 02052), CKMB values of <1, 18, 24 and 72 ng/ml, respectively were determined, indicating good agreement between the two assays.

### Example 13

### Thyroid Stimulating Hormone (TSH) Assay

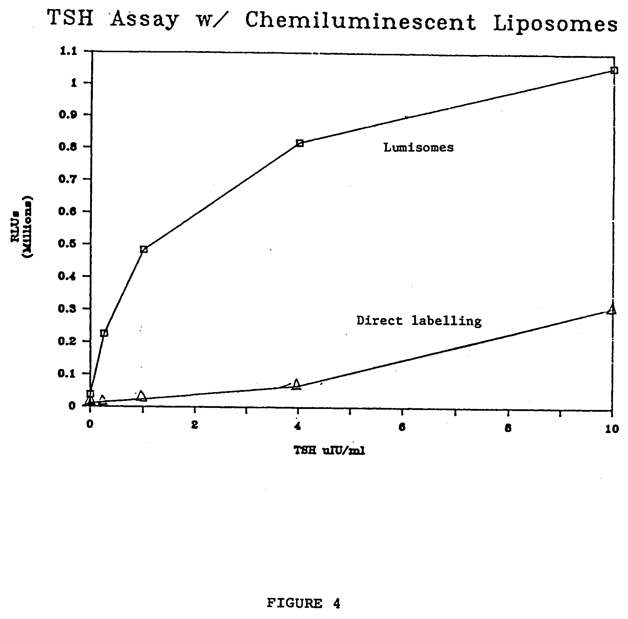

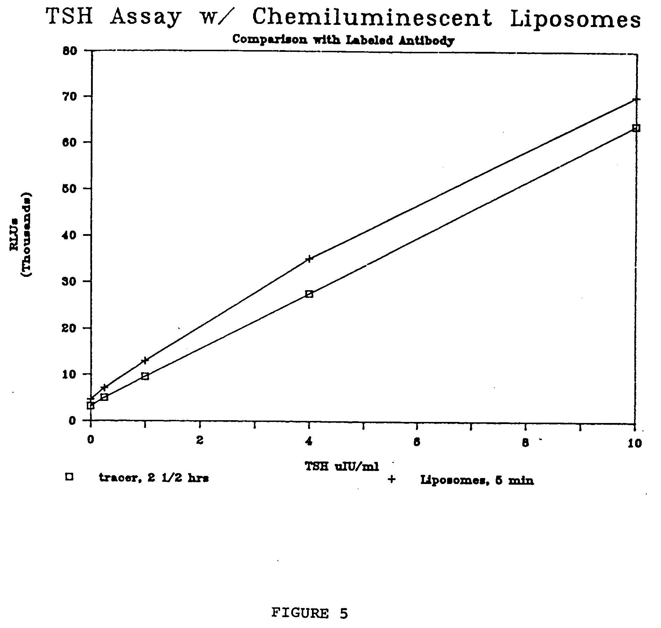

A. Reagent preparation
   Anti-TSH monoclonal antibodies were produced from TSH-immunized mice as described in Example 10 for the preparation of anti-T₄ antibodies. Preparation of DTP-DPPE containing lumisomes loaded with DMAE-ANDS and paramagnetic particles-immobilized anti-TSH was done as described in Example 12. These reagents were diluted in Buffer C.
B. Assay procedure.
   A series of serum-based standards (0.1 ml) with known increasing concentrations of TSH were added to 12 x 75 mm plastic tubes. 0.1 ml of the anti-TSH antibody bearing lumisomes prepared in A containing 8x10⁶ RLU were added and the tubes were then incubated for 2 hours at room temperature. Paramagnetic-particles immobilized anti-TSH antibody as prepared in A was then added (0.5 ml) and the tubes were then incubated for an additional 30 minutes at room temperature. The tubes were then processed and read as described in Example 11.
C. The TSH assay was performed by the same procedure as described in B, except that the anti-TSH antibody bearing lumisomes were replaced by the anti-TSH antibody prepared in A labeled directly with 2′,6′-dimethyl-4′-(N-succinimidyloxycarbonyl)phenyl 10-methyl-acridinium-9-carboxylate methosulfate (DMAE-NHS).
D. The standard curves obtained by the procedures described in B and C using the two types of labels are shown in Figure 4. The results indicate that by using lumisomes encapsulating the hydrophilic acridinium ester analogue as the label, the signal to noise ratio at the low end of the assay is significantly increased. This increase in signal to noise ratio is advantageous because it allows better assay sensitivity, precision and speed.
E. The assay described in B was conducted using shorter incubation times (see Figure 5). It was found that even when the first incubation time was 2.5 minutes and the second incubation time was also 2.5 minutes the sensitivity of the assay was satisfactory. It was not possible to obtain a sufficiently sensitive standard curve using the anti-TSH antibody which had been labeled directly with DMAE-NHS using the short incubation times. Surprisingly, the signal to noise ratio of the short lumisome assay was higher than that of the 2.0 hour/0.5 hour conventional assay (Figure 5).

## Claims

1. A liposome characterised in that it comprises an acridinium ester encapsulated therein.

2. A liposome as claimed in claim 1 wherein the acridinium ester corresponds to the following general formula: 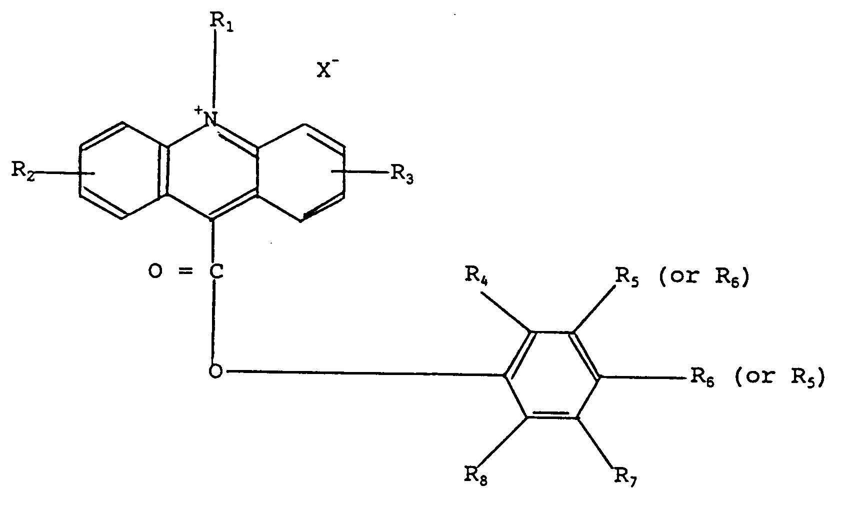 wherein R₁ represents alkyl, alkenyl, alkynyl, aryl or aralkyl, which may contain one or more heteroatoms;
R₂, R₃, R₅ and R₇ independently represent hydrogen, amino, alkoxyl, hydroxyl, -COOH, halide, nitro, -CN, -SO₃H, -NHC(O)R, -C(O)R, -C(O)OR, -C(O)NHR or -SCN; wherein R represents alkyl, alkenyl, alkynyl, aryl or aralkyl, which may contain one or more heteroatoms;
R₄ and R₈ independently represent hydrogen, alkyl, alkenyl, alkynyl, aralkyl or alkoxyl;
R₆ represents -COOH, -R-Iₙ or -Q-R-Iₙ; wherein R represents a diradical derived from alkyl, alkenyl, alkynyl, aryl or aralkyl, which may contain one or more heteroatoms; Q represents -O-, -S-, -NH-, -C(O)-, -SO₃, diazo, -NHC(S)NH-, -NHC(O)NH-, -NHC(O)O-, -NHC(O)-, -C(O)NH- or -NHC(N⁺H₂)-; I represents -SO₃H, -OSO₃H, -PO(OH)₂, -OPO(OH)₂ or -COOH; and n represents an integer of at least 1;
and
X represents an anion.

3. A liposome as claimed in claim 2 wherein R₁ and R may contain up to 24 carbon atoms and/or may contain up to 20 heteroatoms, which may be selected from nitrogen, oxygen, phosphorus and sulfur; R₁ represents C₁-C₁₀ alkyl; and/or wherein R₂, R₃, R₅ and R₇ independently represent hydrogen, amino, -COOH, -CN, hydroxyl, C₁-C₄ alkoxyl, nitro, halide, -SO₃H or -SCN; and/or wherein X represent halide, CH₃SO₄⁻, FSO₃⁻, CF₃SO₃⁻, C₄F₉SO₃⁻ or OSO₂-Ph-CH₃; and/or wherein Q represents -O-, -S-, -NH-, -C(O)-, diazo, -NHC(S)NH-, -NHC(O)NH-, -NHC(O)-, -C(O)NH- or -NHC(N⁺H₂)-; and/or wherein R₄ and R₈ independently represent hydrogen, alkyl, alkenyl, alkynyl or alkoxyl, which may contain up to 8 carbon atoms; and/or wherein R₆ represents -Q-R-Iₙ; and/or wherein I represents -SO₃H, -OSO₃H, -PO(OH)₂, -OPO(OH)₂ or -COOH; and/or wherein n represents an integer of up to 20; and/or wherein X represents an anion.

4. A liposome as claimed in claim 3 wherein R₁ and R may contain up to 10 heteroatoms; and/or wherein R₂, R₃, R₅ and R₇ independently represent hydrogen, C₁-C₄ alkoxyl, nitro, amino or -SO₃H; and/or wherein X represents halide; and/or R₄ and R₈ independently represents C₁-C₄ alkyl; and/or wherein n represents an integer of up to 10.

5. A liposome as claimed in any of claims 2 to 4 wherein X represents bromide, R₁, R₄ and R₈ represent methyl; R₂, R₃, R₅ and R₇ represent hydrogen; and R₆ represents -Q-R-Iₙ wherein Q represents -C(O)- and R-Iₙ is selected from aminomethane sulfonic acid, 7-amino-1, 3-naphthalene disulfonic acid, S-(3-sulfopropyl)cysteine, 2-aminoethyl hydrogen sulfate, 2-aminoethyl phosphonic acid and 2-aminoethyl dihydrogen phosphate.

6. An acridinium ester characterised in that it is as defined in any of claims 2 to 5, provided that the substituent at C4 of the phenoxy group is other than -COOH.

7. A luminescent assay conjugate characterised in that it comprises a liposome as claimed in any of claims 1 to 5 coupled to at least one biologically-active molecule.

8. A conjugate as claimed in claim 7 wherein the biologically-active molecule is selected from antibodies, antigens, haptens and nucleic acid(s).

9. A method for the determination of an analyte charaterised in that it comprises combining a sample fluid, a liposome as claimed in any of claims 1 to 5 or a conjugate as claimed in claim 7 or claim 8 and a first receptor for the analyte, and then determining the amount of liposome label associated with the analyte; or, when the analyte is a member of a specific binding ligand/antiligand pair, detecting the value of the detectable liposome label as compared to the determination of the detectable label of a sample with a known amount of analyte.

10. A method as claimed in claim 9 wherein the analyte is an antigen, a hapten, a DNA probe or a RNA probe and/or the receptor is an antibody.

11. A method as claimed in claim 9 or claim 10 wherein the sample fluid is combined with a solid support comprising a second receptor for the analyte prior to, simultaneously with or after combining the sample fluid with the liposome.

## Patentansprüche

1. Liposom, dadurch gekennzeichnet, daß es einen Acridiniumester darin eingeschlossen enthält.

2. Liposom nach Anspruch 1, wobei der Acridiniumester die nachfolgende allgemeine Formel aufweist: 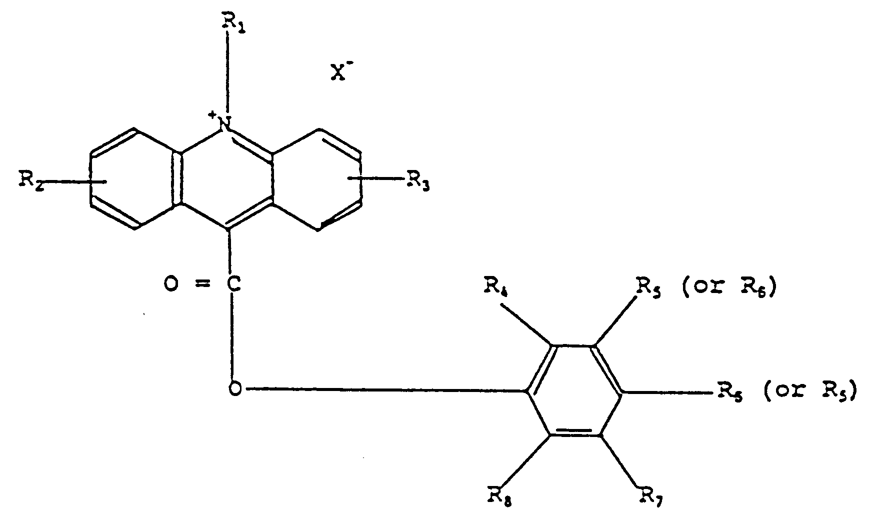 wobei R₁ Alkyl, Alkenyl, Alkynyl, Aryl or Aralkyl ist, die eine oder mehrere Heteroatome enthalten können;
R₂, R₃, R₅ und R₇ sind unabhängig voneinander Wasserstoff, Amino, Alkoxyl, Hydroxyl, -COOH, Halogenid, Nitro, -CN, -SO₃H, -NHC(O)R, -C(O)R, -C(O)OR, -C(O)NHR oder -SCN, wobei R Alkyl, Alkenyl, Alkynyl, Aryl oder Aralkyl ist, die eine oder mehrere Heteroatome enthalten können;
R₄ und R₈ sind unabhängig voneinander Wasserstoff, Alkyl, Alkenyl, Alkynyl, Aralkyl oder Alkoxyl;
R₆ ist -COOH, -R-Iₙ oder -Q-R-Iₙ, wobei R ein Diradikal ist, abgeleitet von Alkyl, Alkenyl, Alkynyl, Aryl oder Aralkyl, die eine oder mehrere Heteroatome enthalten können; Q ist -O-, -S-, -NH-, -C(O)-, -SO₃, Diazo, -NHC(S)NH-, -NHC(O)NH-, -NHC(O)O-, -NHC(O)-, -C(O)NH-oder -NHC(N⁺H₂)-; I ist -SO₃H, -OSO₃H, -PO(OH)₂, -OPO(OH)₂ oder -COOH; und n ist ein Ganzzahliges von wenigstens 1;
und
X ist ein Anion.

3. Liposom nach Anspruch 2, wobei R₁ und R bis zu 24 Kohlenstoffatome enthalten können und/oder bis zu 20 Heteroatome enthalten können, die aus Stickstoff, Sauerstoff, Phosphor und Schwefel ausgewählt werden können; R₁ ist C₁-C₁₀-Alkyl; und/oder wobei R₂, R₃, R₅ und R₇ unabhängig voneinander Wasserstoff, Amino, -COOH, -CN, Hydroxyl, C₁-C₄-Alkoxyl, Nitro, Halogenid, -SO₃H oder -SCN sind; und/oder wobei X ein Halogenid, CH₃SO₄⁻, FSO₃⁻, CF₃SO₃⁻, C₄F₉SO₃⁻ oder OSO₂-Ph-CH₃ ist; und/oder wobei Q -O-, -S-, -NH-, -C(O)-, Diazo, -NHC(S)NH-, -NHC(O)NH-, -NHC(O)-, -C(O)NH- oder -NHC(N⁺H₂)- ist; und/oder wobei R₄ und R₈ unabhängig voneinander Wasserstoff, Alkyl, Alkenyl, Alkynyl oder Alkoxyl sein können, die bis zu 8 Kohlenstoffatome enthalten können; und/oder wobei R₆ -Q-R-Iₙ ist; und/oder wobei I -SO₃H, -OSO₃H, -PO(OH)₂, -OPO(OH)₂ oder -COOH ist; und/oder wobei n ein Ganzzahliges von bis zu 20 ist; und/oder wobei X ein Anion ist.

4. Liposom nach Anspruch 3, wobei R₁ und R bis zu 10 Heteroatome enthalten können; und/oder wobei R₂, R₃, R₅ und R₇ unabhängig voneinander Wasserstoff, C₁-C₄-Alkoxyl, Nitro, Amino oder -SO₃H sind; und/oder wobei X ein Halogenid ist; und/oder R₄ und R₈ unabhängig voneinander C₁-C₄-Alkyl sind; und/oder wobei n ein Ganzzahliges von bis zu 10 ist.

5. Liposom nach einem der Ansprüche 2 bis 4, wobei X ein Bromid ist, R₁, R₄ und R₈ Methyl sind; R₂, R₃, R₅ und R₇ Wasserstoff sind; und R₆ -Q-R-Iₙ ist, wobei Q -C(O)- ist und R-Iₙ aus Aminomethansulfonsäure, 7-Amino-1,3-naphthalendisulfonsäure, S-(3-Sulfopropyl)-cystein, 2-Aminoethylhydrogensulfat, 2-Aminoethylphosphonsäure und 2-Aminoethyldihydrogenphosphat ausgewählt ist.

6. Acridiniumester, dadurch gekennzeichnet, daß er wie in einem der Ansprüche 2 bis 5 definiert ist, mit der Einschränkung, daß der Substituent am C4 der Phenoxygruppe nicht -COOH ist.

7. Lumineszenzassay-Konjugat, dadurch gekennzeichnet, daß es ein Liposom nach einem der Ansprüche 1 bis 5 gekoppelt mit wenigstens einem biologisch aktiven Molekül umfaßt.

8. Konjugat nach Anspruch 7, wobei das biologisch aktive Molekül aus Antikörpern, Antigenen, Haptenen und Nucleinsäure(n) ausgewählt ist.

9. Verfahren zur Bestimmung eines Analyten, dadurch gekennzeichnet, daß es das Kombinieren einer Probenflüssigkeit, eines Liposoms nach einem der Ansprüche 1 bis 5 oder eines Konjugats nach Anspruch 7 oder Anspruch 8 und eines ersten Rezeptors für den Analyten und das anschließende Bestimmen der mit dem Analyten assoziierten Liposomenmarkierungsmenge umfaßt; oder, wenn der Analyt ein Element eines spezifischen Bindungsliganden/Antiliganden-Paares ist, Nachweisen des Wertes der nachweisbaren Liposomenmarkierung im Vergleich zur Bestimmung der nachweisbaren Markierung einer Probe mit einer bekannten Analytmenge.

10. Verfahren nach Anspruch 9, wobei der Analyt ein Antigen, ein Hapten, eine DNA-Probe oder eine RNA-Probe und/oder der Rezeptor ein Antikörper ist.

11. Verfahren nach Anspruch 9 oder 10, wobei die Probenflüssigkeit mit einem einen zweiten Rezeptor für den Analyten umfassenden Festträger vor, gleichzeitig mit oder nach dem Vereinigen der Probenflüssigkeit mit dem Liposom kombiniert wird.

## Revendications

1. Un liposome caractérisé en ce qu'il contient un ester d'acridinium qui y est encapsulé.

2. Un liposome tel que revendiqué dans la revendication 1, dans lequel l'ester d'acridinium correspond à la formule générale suivante : 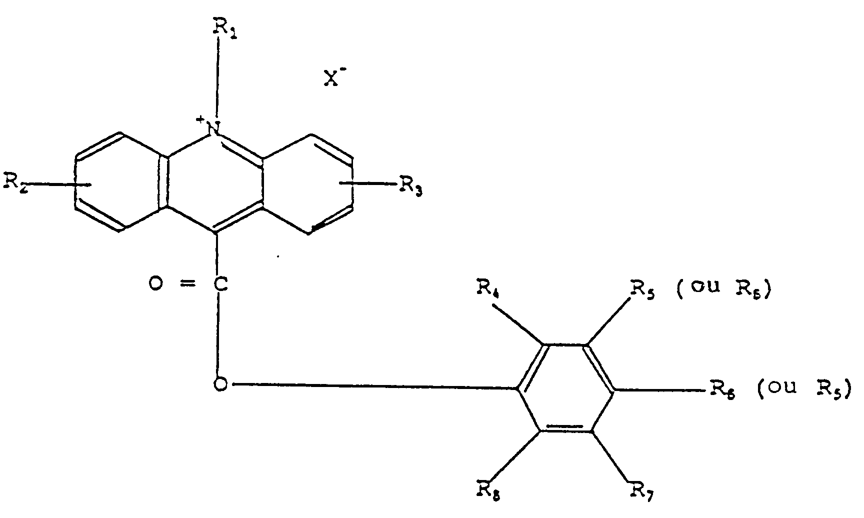 dans laquelle R₁ représente un groupe alkyle, alcényle, alcynyle, aryle ou aralkyle, qui peut contenir un ou plusieurs hétéroatomes ;
R₂, R₃, R₅ et R₇ représentent indépendamment l'hydrogène, un groupe amino, alcoxy, hydroxyle, -COOH, halogénure, nitro, -CN, -SO₃H, -NHC(O)R, -C(O)R, -C(O)OR, -C(O)NHR ou -SCN ; où R représente un groupe alkyle, alcényle, alcynyle, aryle ou aralkyle, qui peut contenir un ou plusieurs hétéroatomes ;
R₄ et R₈ représentent indépendamment l'hydrogène, un groupe alkyle, alcényle, alcynyle, aralkyle ou alcoxy ;
R₆ représente -COOH, -R-Iₙ ou -Q-R-Iₙ ; où R représente un diradical dérivé d'un groupe alkyle, alcényle, alcynyle, aryle ou aralkyle, qui peut contenir un ou plusieurs hétéroatomes ; Q représente -O-, -S-, -NH-, -C(O)-, -SO₃, un groupe diazo, -NHC(S)NH-, -NHC(O)NH-, -NHC(O)O-, -NHC(O)-, -C(O)NH- ou -NHC(N⁺H₂)- ; I représente -SO₃H, -OSO₃H, -PO(OH)₂, -OPO(OH)₂ ou -COOH ; et n représente un nombre entier égal ou au moins 1 ; et
X représente un anion.

3. Un liposome tel que revendiqué dans la revendication 2, dans lequel R₁ et R peuvent contenir jusqu'à 24 atomes de carbone et/ou peuvent contenir jusqu'à 20 hétéroatomes qui peuvent être choisis parmi l'azote, l'oxygène, le phosphore et le soufre ; R₁ représente un groupe alkyle en C₁-C₁₀ ; et/ou dans lequel R₂, R₃, R₅ et R₇ représentent indépendamment l'hydrogène, un groupe amino, -COOH, -CN, hydroxyle, alcoxy en C₁-C₄, nitro, halogénure, -SO₃H ou -SCN ; et/ou dans lequel X représente un halogénure, CH₃SO₄⁻, FSO₃⁻, CF₃SO₃⁻, C₄F₉SO₃⁻ ou OSO₂-Ph-CH₃ ; et/ou dans lequel Q représente -O-, -S-, -NH-, -C(O)-, un groupe diazo, -NHC(S)NH-, -NHC(O)NH-, -NHC(O)-, -C(O)NH- ou -NHC(N⁺H₂)- ; et/ou dans lequel R₄ et R₈ représentent indépendamment l'hydrogène, un groupe alkyle, alcényle, alcynyle ou alcoxy, qui peut contenir jusqu'à 8 atomes de carbone ; et/ou dans lequel R₆ représente -Q-R-Iₙ ; et/ou dans lequel I représente -SO₃H, -OSO₃H, -PO(OH)₂, -OPO(OH)₂ ou -COOH ; et/ou dans lequel n représente un nombre entier égal ou inférieur à 20 ; et/ou dans lequel X représente un anion.

4. Un liposome tel que revendiqué dans la revendication 3, dans lequel R₁ et R peuvent contenir jusqu'à 10 hétéroatomes ; et/ou dans lequel R₂, R₃, R₅ et R₇ représentent indépendamment l'hydrogène, un groupe alcoxy en C₁-C₄, nitro, amino ou -SO₃H ; et/ou dans lequel X représente un halogénure ; et/ou R₄ et R₈ représentent indépendamment un groupe alkyle en C₁-C₄ ; et/ou dans lequel n représente un nombre entier égal ou inférieur à 10.

5. Un liposome tel que revendiqué dans l'une quelconque des revendications 2 à 4, dans lequel X représente le bromure, R₁, R₄ et R₈ représentent le groupe méthyle ; R₂, R₃, R₅ et R₇ représentent l'hydrogène ; et R₆ représente -Q-R-I ₙ où Q représente -C(O)- et R-Iₙ est choisi parmi l'acide aminométhanesulfonique, l'acide 7-aminonaphtalène-1,3-disulfonique, la S-(3-sulfopropyl)cystéine, l'hydrogénosulfate de 2-aminoéthyle, l'acide 2-aminoéthylphosphonique et le dihydrogénophosphate de 2-aminoéthyle.

6. Un ester d'acridinium caractérisé en ce qu'il est tel que défini dans l'une quelconque des revendications 2 à 5, à condition que le substituant à la position C₄ du groupe phénoxy soit autre chose que -COOH.

7. Un conjugué pour analyse par luminescence, caractérisé en ce qu'il comprend un liposome tel que revendiqué dans l'une quelconque des revendications 1 à 5 couplé à au moins une molécule biologiquement active.

8. Un conjugué tel que revendiqué dans la revendication 7, dans lequel la molécule biologiquement active est choisie parmi des anticorps, des antigènes, des haptènes et un ou plusieurs acides nucléiques.

9. Une méthode pour la détermination d'un analyte, caractérisé en ce qu'elle consiste à combiner un échantillon de fluide, un liposome tel que revendiqué dans l'une quelconque des revendications 1 à 5 ou un conjugué tel que revendiqué dans la revendication 7 ou la revendication 8 et un premier récepteur pour l'analyte, puis à déterminer la quantité de marqueur liposomal associé à l'analyte ; ou, lorsque l'analyte est un membre d' une paire ligand/antiligand à liaison spécifique, à détecter la valeur du marqueur liposomal détectable par comparaison avec la détermination du marqueur détectable d'un échantillon contenant une quantité connue d'analyte.

10. Une méthode telle que revendiquée dans la revendication 9, dans laquelle l'analyte est un antigène, un haptène, une sonde ADN ou une sonde ARN et/ou le récepteur est un anticorps.

11. Une méthode telle que revendiquée dans la revendication 9 ou la revendication 10, dans laquelle l'échantillon de fluide est combiné à un support solide comprenant un second récepteur pour l'analyte avant, pendant ou après la combinaison de l'échantillon de fluide avec le liposome.
